# EUROPÄISCHE PATENTSCHRIFT

(11) **EP 3 730 162 B1**
(45) Veröffentlichungstag und Bekanntmachung des Hinweises auf die Patenterteilung: **07.12.2022**
(21) Anmeldenummer: 19171363.5
(22) Anmeldetag: 26.04.2019
(51) Int. Cl.: A61L 27/30, A61L 27/42, A61L 31/08, A61L 31/12

(54) **DUKTILE BESCHICHTUNG FÜR EINE IMPLANTATKOMPONENTE**
DUCTILE COATING FOR AN IMPLANT COMPONENT
REVÊTEMENT DUCTILE POUR UN COMPOSANT D'IMPLANT

(43) Veröffentlichungstag der Anmeldung: 28.10.2020
(73) Patentinhaber: Waldemar Link GmbH & Co. KG, 22339 Hamburg (DE)
(72) Erfinder: LINK, Helmut D., 22397 Hamburg (DE); CSASZAR, Richard, 23795 Bad Segeberg (DE)
(74) Vertreter: AWA Denmark A/S

(56) Entgegenhaltungen:
- WO-A1-2015/150186
- WO-A2-2009/095705
- LIN XIAO ET AL: "Orthopedic implant biomaterials with both osteogenic and anti-infection capacities and associatedin vivoevaluation methods", NANOMEDICINE: NANOTECHNOLOGY, BIOLOGY AND MEDICINE, ELSEVIER, NL, Bd. 13, Nr. 1, 20. August 2016 (2016-08-20), Seiten 123-142, XP029879757, ISSN: 1549-9634, DOI: 10.1016/J.NANO.2016.08.003
- RICHARD KUEHL ET AL: "Preventing Implant-Associated Infections by Silver Coating", ANTIMICROBIAL AGENTS AND CHEMOTHERAPY, Bd. 60, Nr. 4, 1. April 2016 (2016-04-01), Seiten 2467-2475, XP055633703, US ISSN: 0066-4804, DOI: 10.1128/AAC.02934-15

## Beschreibung

### GEBIET DER ERFINDUNG

Die Erfindung betrifft eine Implantatkomponente mit eine TiNb-Ag Beschichtung, ein Verfahren zum Herstellen einer Implantatkomponente durch Auftragen dieser Beschichtung sowie eine Verwendung dieser Beschichtung für die Herstellung einer Implantatkomponente.

### STAND DER TECHNIK

Einer der Gründe, die ein Versagen eines Implantats in situ hervorrufen können, ist eine Infektion mit Erregern. Solche Erreger sind vor allem Staphylokokken, wie zum Beispiel Staphylococcus epidermidis, welche die Haut und Schleimhäute von Menschen besiedeln. Zudem hat unter anderem beispielsweise der Erreger Staphylococcus epidermidis die Fähigkeit, sich mit Hilfe eines Biofilms auf einer Implantatoberfläche anzusiedeln. Dieser Biofilm schützt dabei so einen Erreger vor Antibiotika, Phagozyten sowie anderen Immunantworten des Körpers.

Studien weisen darauf hin, dass zum Beispiel Staphylococcus epidermidis nach der Implantation von Implantaten eine häufige Ursache für postoperative Infektionen ist. Bei der Diagnose einer postoperativen Infektion wird im Allgemeinen zunächst versucht, diese durch Wirkstoffe, wie zum Beispiel Antibiotika, zurückzudrängen. Zeigt dieses Vorgehen keinen Erfolg, kann es notwendig werden, das Implantat wieder zu explantieren. Bei der Entnahme des Implantats wird so weit wie möglich auch der Infektionsherd entfernt. Zwar führt dies zu einer effektiveren Behandlung der Infektion, hat aber eine verminderte Mobilität des Patienten zur Folge.

Im Fall einer Infektion mit Staphylococcus epidermidis kommt erschwerend hinzu, dass dieser Keim regelmäßig Antibiotikaresistenzen aufweist (80% nach Takizawa et al. in SPINE Volume 42, Nr. 7, pp 525-530). Nach Takizawa gibt es im Bereich der Wirbelsäulenchirurgie Anzeichen, dass die als Methicillin-resistente Staphylococcus epidermidis (MRSE) bekannten Erreger wegen ihrer geringeren Virulenz anfangs weniger Infektionszeichen zeigen und damit das Risiko in sich bergen, dass eine Infektion erst spät erkannt wird. Dies kann für einen Patienten im schlimmsten Fall postoperativ zu einer höheren Morbidität führen als präoperativ, insbesondere wenn die Infektion die weitere Verwendung eines Implantats ausschließt.

Um solche Komplikationen zu umgehen, ist es somit erstrebenswert, bereits der Ansiedlung von Erregern entgegenzuwirken. So schlägt die US 2009/0198343 A1 vor, eine Beschichtung für ein künstliches Gelenk, die für die Laufflächen von Metallpaarungen vorgesehen ist, mit einer antimikrobiellen Wirkung zu versehen. Um dies zu erreichen, wird in der US 2009/0198343 A1 eine Chromnitridbeschichtung durch Silber ergänzt, indem die Reibfläche des Implantats abwechselnd mit Chromnitrid und Silber beschichtet wird. Jedoch ist Chromnitrid als sensibilisierend eingestuft, sodass nach Implantation die Gefahr besteht, allergische Reaktionen hervorzurufen. Zudem ist die in der US 2009/0198343 A1 offenbarte Beschichtung nur sehr eingeschränkt gegen eine Ansiedlung von Staphylococcus epidermidis wirksam. In einer Literaturübersicht vergleichen Lin Xiao et al. ("Orthopedic implant biomaterials with both osteogenic and anti-infection capacities and associated in vivo evaluation methods. Nanomedicine: NBM 2017, Vol. 13, pages 123-142, ISSN 1549-9634) antibakterielle und osteogene Eigenschaften von verschiedenen Biomaterialien. Die WO 2015/150186 A1 betrifft eine Implantatkomponente, die einen Verbindungsabschnitt aufweist, der zumindest teilweise mit einer TiNb-Beschichtung beschichtet ist und bildet die Grundlage für die zweiteilige Form des ersten Anspruchs.

Hinzu kommt, dass es bei manchen Implantatkomponenten wünschenswert ist, sie vor einem Einbringen in den Körper eines Patienten durch Biegen anpassen zu können. Mit anderen Worten sind diese Implantatkomponenten plastisch verformbar gestaltet, sodass sie an die Umgebung der Implantatkomponente oder die Lage anderer Implantatkomponenten anpassbar sind. Diese Eigenschaften sind insbesondere bei Stangen im Wirbelsäulenbereich und Knochenplatten zur Behandlung von Frakturen vorteilhaft. Gerade Nitridbeschichtungen sind hierfür jedoch nur eingeschränkt verwendbar, da sie nicht nur relativ hart sondern auch relativ spröde sind. Eine plastische Verformung einer mit einer Nitridbeschichtung versehenen Implantatkomponente kann deswegen zu Schäden an der Beschichtung führen. Als Folge hiervon können vermehrt Metallionen, Metalloxide, Metallorganophosphate und kleine Metallpartikel freigesetzt werden und führen möglicherweise zu Schmerzen, einer aseptischen Lockerung der Implantatkomponente oder negativen Folgewirkungen für das umliegende Gewebe. Auch kann es zu allergischen Reaktionen kommen, die ebenfalls eine Revision des Implantats erforderlich machen können.

### ZUSAMMENFASSUNG DER ERFINDUNG

Folglich war es Aufgabe der vorliegenden Erfindung, eine Beschichtung für eine Implantatoberfläche bereitzustellen, die einer Infektion in situ vorbeugt. Insbesondere war es Aufgabe der Erfindung, eine Beschichtung für eine Implantatoberfläche bereitzustellen, die der Ansiedlung von Erregern, insbesondere von Staphylococcus epidermidis, entgegenwirkt. Auch war es Ziel, dass die beschichtete Implantatoberfläche keine Allergien oder Überempfindlichkeiten beim Patienten hervorruft. Weiterhin sollte die beschichtete Implantatoberfläche etwaigen mechanischen Einflüssen, die insbesondere durch plastische Verformung der Implantatkomponente entstehen, widerstehen können.

Angesichts dieser Aufgaben wird die in den Ansprüchen definierte Implantatkomponente, die zumindest abschnittsweise mit einer Beschichtung beschichtet ist, ein Verfahren zum Herstellen einer Implantatkomponente mit dieser Beschichtung und eine Verwendung dieser Beschichtung für die Herstellung einer Implantatkomponente bereitgestellt

Die Erfindung betrifft eine Implantatkomponente, die zumindest abschnittsweise mit einer Beschichtung beschichtet ist, wobei die Beschichtung eine TiNb-Beschichtung ist, die einem At%-Anteil Ti und einen At%-Anteil Nb aufweist, dadurch gekennzeichnet, dass die Beschichtung zudem einen At%- Anteil von 1-25 At% Ag aufweist und dass die Implantatkomponente mit der Beschichtung plastisch verformbar ist.

Die Erfindung betrifft weiterhin ein Verfahren zum Herstellen der Implantatkomponente, das die Schritte umfasst: Bereitstellen einer zu beschichtenden Implantatkomponente in einer Beschichtungskammer; Bereitstellen von mindestens einem Target, sodass bei einem Verdampfen ein vorbestimmtes At%-Verhältnis von Titan, Niob und Silber erzeugt wird; Bereitstellen einer inerten Atmosphäre; Verdampfen des mindestens einen Targets; und gleichzeitiges Beschichten der Implantatkomponente mit dem verdampften Metall des mindestens einen Targets, und plastisches Verformen der beschichteten Implantatkomponente vor der Implantation.

Die Erfindung betrifft weiterhin die Verwendung der Beschichtung, wobei die Beschichtung eine TiNb-Beschichtung ist, die einem At%-Anteil Ti und einen At%-Anteil Nb aufweist, und wobei die Beschichtung zudem einen At%- Anteil von 1-25 At% Ag aufweist, für die Herstellung einer Implantatkomponente die plastisch verformbar ist.

Die Beschichtung ist für eine Implantatkomponente, insbesondere eine Wirbelsäulenimplantatkomponente, vorgesehen und ist eine TiNb-Beschichtung, die neben einem At%-Anteil Ti und einem At%-Anteil Nb einen At%- Anteil von 1-25 At% Ag aufweist.

Der bei dieser Beschichtung vorliegende Silberanteil ist in der Lage, gegen eine durch Erreger hervorgerufene Infektion vorbeugen. Diesbezüglich hat sich insbesondere eine antimikrobielle Wirkung gegen Staphylococcus epidermidis gezeigt. Dabei ist dem Fachmann verständlich, dass es sich bei der vorliegenden Beschichtung um eine über ein technisches, maschinelles Verfahren aufgebrachte Beschichtung handelt, die bei der Herstellung des Implantats erzeugt wird.

Weiterhin führt der At%-Anteil Ag, d. h. der Silberanteil, in dem oben definierten Bereich zu keiner nennenswerten Verminderung der mechanischen Widerstandsfähigkeit der Beschichtung, sodass diese für die bei der Implantation auftretenden mechanischen Kontaktkräfte ausreicht. Es wird vermutet, dass die mechanische Widerstandsfähigkeit aufgrund eines beschränkten Einflusses des Silberanteils auf die Duktilität auf einem derartigen Niveau bleibt. Damit wird dagegen vorgebeugt, dass das unter der Beschichtung befindliche Implantatmaterial beziehungsweise Basismaterial des Implantats mit Körpergewebe eines Patienten in Kontakt kommt und dabei möglicherweise zu einer Überempfindlichkeit führt.

Durch ihre Widerstandsfähigkeit und Duktilität ist die Titan-Niob-Beschichtung mit Silberanteil, d. h. TiNb-Ag-Beschichtung, insbesondere für strukturelle Implantate geeignet, die Teile des Skeletts unterstützen oder ersetzen, wenn sie in den Körper eines Patienten eingebracht worden sind. Ein Beispiel hierfür sind stangenförmige Verbindungselemente, die im Wirbelsäulenbereich bei einer Spondylodese eingesetzt werden, um zwei Wirbelkörper relativ zueinander zu fixieren. Derartige Verbindungselemente werden regelmäßig vor der Fixierung im Körper plastisch verformt, um die Wirbelkörper in einer vorbestimmten Lage zueinander zu fixieren. Aufgrund der duktilen Eigenschaften bleibt die vorliegende Beschichtung bei plastischer Verformung strukturell erhalten, sodass im Körper des Patienten einem Austreten von Legierungsbestandteile vorgebeugt wird, die anderweitig zu Überempfindlichkeiten führen könnten. Mit anderen Worten entstehen bei der Beschichtung durch die plastische Verformung im Wesentlichen keine Risse oder Abplatzungen.

Dabei ist es von Vorteil, wenn die beschichtete Implantatkomponente im Körper des Patienten keiner Reibung mit einer anderen Implantatkomponente ausgesetzt ist, die durch eine wechselnde oder schwellende Belastung der Implantatkomponenten ausgesetzt ist. Mit andere Worten sollte zwischen der beschichteten Implantatkomponente und einer benachbarten Implantatkomponente im Wesentlichen keine Relativbewegung auftreten, wie zum Beispiel bei Gelenkflächen eines Gelenkersatzes der Fall.

Es ist jedoch anzumerken, dass die Beschichtung auch vorteilhaft bei anderen Implantatkomponenten, eingesetzt werden kann, die einer starken elastischen oder plastischen Biegung ausgesetzt sind. Hierzu gehören zum Beispiel Knochenplatten, die zur Heilung von Frakturen eingesetzt werden und wie die oben genannten stangenförmigen Verbindungselemente im Wirbelsäulenbereich unmittelbar vor ihrer endgültigen Implantation durch plastische Verformung an die anatomische Umgebung angepasst werden. Ein weiteres Beispiel, für das die vorliegende Beschichtung besonders vorteilhaft ist, sind biegsame Laschen, wie sie beispielsweise bei Beckenteilersatzprothesen zum Einsatz kommen.

Bei einer bevorzugten Ausführungsform weist die Beschichtung 1,5-15 At% Ag, 1,5-5 At% Ag oder in etwa 2 At% Ag auf.

Diese bevorzugten Silberanteile in der Beschichtung erfüllen die obigen Anforderungen. In Bezug auf die Härte gilt, dass, je niedriger der Silberanteil ist, desto höher die Härte ist. Allerdings wirkt sich eine Verminderung der Härte, wie oben beschrieben, nicht wesentlich auf die Widerstandsfähigkeit und Duktilität der Beschichtung aus.

Bei einer weiteren bevorzugten Ausführungsform weist die Beschichtung 5-40 At% Nb, 10-30 At% Nb, 15-25 At% Nb oder in etwa 18 At% Nb auf.

Diese bevorzugten Anteile an Niob beugen gegen überempfindliche Reaktionen im Körper des Patienten vor und sorgen zudem für die notwendige Duktilität, um bei den während eines Eingriffs auftretenden plastischen Verformungen im Wesentlichen keinen Schaden zu nehmen.

Wie oben beschrieben, werden neben dem Ag-Anteil und dem Nb-Anteil die verbleibenden Anteile im Wesentlichen durch Ti belegt. Im Wesentlichen bezieht sich in diesem Zusammenhang darauf, dass produktionsbedingte Verunreinigungen durch andere Verbindung vorliegen können, die jedoch einen At%-Anteil von 3%, 2% oder 1% nicht überschreiten. Vorzugsweise hat Ti bei der vorliegenden Beschichtung den höchsten At%-Anteil. Insbesondere beträgt der At%-Anteil an Titan vorzugsweise 65-90 At%, 75-85 At% oder in etwa 80 At%.

Bei einer Ausführungsform sind auf der Beschichtungsoberfläche Ag und TiNb nebeneinanderliegend ausgebildet.

Hierdurch kann der Ag-Anteil seine infektionshemmende Wirkung besser entfalten. Um diesem an der Oberfläche nebeneinander liegenden Aufbau der Beschichtung und damit direkten Kontakt mit dem Gewebe oder Flüssigkeiten des Patienten zu erreichen, werden die beiden Beschichtungskomponenten vorzugsweise gleichzeitig aufgebracht. Durch dieses gleichzeitige Aufbringen liegen die Beschichtungskomponenten zudem im Wesentlichen auch gleichmäßig verteilt auf der Implantatoberfläche vor.

Bei einer bevorzugten Ausführungsform weist die Beschichtung eine Dicke von 2,5-6 µm, 3,5-5,5 µm oder in etwa 4,5 µm auf.

Es wurde festgestellt, dass bei diesen Beschichtungsstärken zumindest eine durchgehende Beschädigung der Beschichtung vermieden wird. Dabei ist eine dickere Beschichtung tendenziell von Vorteil. Eine über diese Werte hinausgehende Dicke bringt hingegen keine wesentlichen Verbesserungen, sondern kann sogar eine Inhomogenität und Delamination der Beschichtung begünstigen. Es wird vermutet, dass die mechanische Widerstandsfähigkeit der Beschichtung bei den angegebenen Dicken auch dadurch bedingt ist, dass keine in der Dickenrichtung durchgehenden Abschnitte aus Silber entstehen. Mit anderen Worten liegt durch die dreidimensionale heterogene Struktur der TiNb-Ag Beschichtung eine im Prinzip durchgehende TiNb-Beschichtung vor.

Bei einer Ausführungsform liegt die TiNb-Beschichtung im Wesentlichen als nicht stöchiometrische TiNb-Schicht vor.

Eine solche Schicht bildet zusammen mit dem Silberanteil eine besonders gleichmäßige inerte Schicht mit antimikrobieller Wirkung aus und wirkt damit nach Implantation des Implantats sowohl einer Überempfindlichkeit als auch einer Infektion auf effektive Weise vor.

Erfindungsgemäß wird eine Implantatkomponente, insbesondere von einem Wirbelsäulenimplantat, bereitgestellt, die zumindest abschnittsweise mit der Beschichtung beschichtet ist, wobei die Implantatkomponente mit der Beschichtung plastisch verformbar ist.

Wie oben beschrieben kann es insbesondere zu Infektionen kommen, die dadurch hervorgerufen werden, dass das Implantat vor der Implantation der Umgebung ausgesetzt wurde. Diesbezüglich konnte festgestellt werden, dass besonders im Wirbelsäulenbereich solche Infektionen häufig auf den Erreger Staphylococcus epidermidis zurückzuführen sind. Deswegen ist die vorbeugende Wirkung einer erfindungsgemäßen Beschichtung insbesondere hier wirkungsvoll.

Es ist hervorzuheben, dass die durch Biegung hervorgerufenen Verschiebungen des Beschichtungsgefüges im Wesentlichen keine Auswirkungen auf die infektionshemmende Wirkung der Beschichtung haben.

Bei einer bevorzugten Ausführungsform ist die Implantatkomponente eine Komponente eines Spondylodeseimplantats und insbesondere eine Verbindungsstange eines solchen Implantats ist.

Bei einem Spondylodeseimplantat werden besonders bevorzugt Verbindungsstangen mit der vorliegenden Beschichtung versehen, welche mindestens zwei Wirbelkörper zur Versteifung verbinden.

Bei einer besonders bevorzugten Ausführungsform weist die Oberfläche des beschichteten Abschnitts TiNb mit darin eingebetteten Ag-Inseln auf.

Wie oben beschrieben, kann durch eine solche Verteilung von TiNb und Silber in der Beschichtung eine im Wesentlichen durchgehende TiNb-Beschichtung bereitgestellt werden, da sich die eingebetteten Ag-Inseln im Allgemeinen nicht durch die gesamte Beschichtungsdicke erstrecken. Damit wird eine ausreichende mechanische Widerstandsfähigkeit bzw. Härte einer solchen Beschichtung erreicht, da TiNb in der Beschichtung eine höhere Härte als Ag aufweist.

Bei einer weiteren bevorzugten Ausführungsform weist die zu beschichtende Implantatkomponente eine Titanlegierung auf und besteht bevorzugt aus dieser.

Zum einen haftet die TiNb-Ag Beschichtung aufgrund des TiNb-Anteils besonders gut auf einer Titanlegierung so einer Implantatkomponente, was gegen ein Ablösen der Beschichtung vorbeugt. Zum anderen reduziert eine zu beschichtende Implantatkomponente aus einer Titanlegierung das Risiko einer Überempfindlichkeit weiter, da eine solche Überempfindlichkeit selbst bei einer Beschädigung der Beschichtung aufgrund der Biokompatibilität von Titan unwahrscheinlich ist. Die Anhaftung der Beschichtung kann bei dieser Ausführungsform noch weiter verbessert werden, indem vor der TiNb-Ag Beschichtung eine im Wesentlichen nur Ti enthaltene Beschichtung als eine Art haftvermittelnde Beschichtung aufgebracht wird.

Weiterhin wird erfindungsgemäß ein Verfahren zum Herstellen einer Implantatkomponente mit einer oben beschriebenen Beschichtung bereitgestellt, das als Schritte ein Bereitstellen einer zu beschichtenden Implantatkomponente in einer Beschichtungskammer, insbesondere einer Implantatkomponente für ein Wirbelsäulenimplantat; Bereitstellen von mindestens einem Target, sodass bei einem Verdampfen ein vorbestimmtes At%-Verhältnis von Titan, Niob und Silber erzeugt wird; Bereitstellen einer inerten Atmosphäre; Verdampfen des mindestens einen Targets; und gleichzeitiges Beschichten der Implantatkomponente mit dem verdampften Metall des mindestens einen Targets , und plastisches Verformen der beschichteten Implantatkomponente vor der Implantation umfasst.

Dieses Verfahren ermöglicht ein gleichzeitiges Beschichten mit Titan, Niob und Silber zum Ausbilden der TiNb-Ag Beschichtung. Dabei wird durch das gleichzeitige Beschichten sichergestellt, dass an der Oberfläche der Beschichtung Silber exponiert ist und sich damit im implantierten Zustand der Implantatkomponente der infektionshemmende Effekt der Beschichtung entfalten kann. Zudem kann durch eine Wahl der Anzahl der jeweiligen Targets der Beschichtungskomponenten der At%-Anteil der Beschichtungskomponenten zumindest in seiner Größenordnung auf die gewünschte Zusammensetzung der Beschichtung eingestellt werden. Dabei entspricht das bei dem Verdampfen erzeugte At%-Verhältnis der Beschichtungskomponenten im Wesentlichen dem angestrebten At%-Verhältnis der auf die Implantatkomponenten aufgebrachten Beschichtung.

Alternativ oder zusätzlich zu der Einstellung der Zusammensetzung der Beschichtung über die Anzahl der jeweiligen Targets kann mindestens ein Target, das ein vorbestimmtes Verhältnis von Titan und Silber aufweist, bereitgestellt werden. Vorzugsweise wird allerdings ausschließlich ein oder mehrere derartige Targets mit entsprechend der gewünschten Zusammensetzung der Beschichtung vorbestimmten At%-Anteilen an Titan, Niob und vorzugsweise Silber verwendet.

Bei einer bevorzugten Ausführungsform wird das Verdampfen des mindestens einen Targets durch Lichtbogenverdampfen ausgeführt. Dabei beträgt die an den Targets angelegte Spannung 15-30 V oder 20-25 V und der angelegte Strom 40-70 A.

Diese Einstellbereiche der Spannung und des Stroms ermöglichen, genauso wie die oben genannte Anzahl der Targets der jeweiligen Beschichtungskomponenten, eine Einstellungsmöglichkeit der Beschichtungszusammensetzung. Dabei kann insbesondere das durch die Wahl der Anzahl der Targets getroffene Verhältnis noch feiner eingestellt werden.

Bei einer weiteren Ausführungsform wird nach dem Bereitstellen des zu beschichtenden Implantats in der Beschichtungskammer eine Reinigung der zu beschichtenden Implantatoberfläche durch eine Glimmentladung unter einer Wasserstoffatmosphäre ausgeführt.

Dieser Reinigungsschritt hat den Vorteil, dass etwaige an der Oberfläche des zu beschichtenden Implantats vorliegende organische Rückstände entfernt werden und so die Haftung der Beschichtung auf dem Implantat verbessert wird.

Bei einer Ausführungsform wird nach dem Einbringen des zu beschichtenden Implantats in die Beschichtungskammer eine Reinigung der zu beschichtenden Implantatoberfläche durch Beschießen der Implantatoberfläche mit hochenergetischen Ionen unter einer inerten Atmosphäre durchgeführt.

Hierdurch wird eine an der Implantatoberfläche vorhandene Oxidschicht entfernt, die anderweitig die Haftung der Beschichtung an dem Implantat reduzieren würde.

Eine derartige Oxidschicht bildet sich zum Beispiel bei Implantaten aus Titanlegierungen und kann insbesondere durch Beschießen mit Argon- und Titanionen durch den Verfahrensschritt dieser Ausführungsform entfernt werden. Die Titanionen können dabei vorzugsweise über ein für die Beschichtung zu verwendendes Ti-Target erzeugt werden. Dabei wirkt die inerte Atmosphäre, wie zum Beispiel eine Argonatmosphäre, einer erneuten Ausbildung einer Oxidschicht entgegen.

Das Herstellungsverfahrens umfasst den Schritt eines plastischen Verformens der beschichteten Implantatkomponente vor der Implantation.

Durch diese plastische Verformung kann die nunmehr beschichtete Implantatkomponente für das und vor dem Einbringen in den Körper des Patienten bezüglich ihrer Form vorbereitet werden.

Des Weiteren wird eine Verwendung (nicht Teil der beanspruchten Erfindung) der oben beschriebenen Beschichtung zum Vorbeugen gegen einen Biofilm auf einem Implantat, insbesondere einem Wirbelsäulenimplantat, bereitgestellt.

Eine derartige Verwendung der Beschichtung ist, wie oben bereits beschrieben, besonders vorteilhaft.

### AUSFÜHRLICHE BESCHREIBUNG BEVORZUGTER AUSFÜHRUNGSFORMEN

Wie oben bereits erwähnt ist unter einer Beschichtung im Rahmen der vorliegenden Erfindung eine durch ein technisches Verfahren aufgebrachte Beschichtung zu verstehen. Beispiele solcher technischer Verfahren sind die chemische Gasphasenabscheidung (CVD - Chemical Vapor Deposition), physikalische Gasphasenabscheidung (PVD - Physical Vapor Deposition) oder galvanische Beschichtungsverfahren.

Wie oben beschrieben umfasst eine erfindungsgemäße Beschichtung eine Mischung aus einer Titan-Niob-Beschichtung, in die Silber eingelagert ist (TiNb-Ag Beschichtung). Mit anderen Worten weist die Beschichtung mindestens eine einzelne Schicht einer Titan-Niob-Beschichtung auf, in der Silber eingebettet ist. Dabei liegt das Silber insbesondere in Form von Silberinseln vor, d. h. Silber beziehungsweise Silberatome sind neben dem TiNb-Gitter angeordnet.

Aufgrund der Größe der Silberatome wird davon ausgegangen, dass, wenn überhaupt, nur ein geringer Anteil des Silbers interstitiell in dem TiNb-Gitter angeordnet ist. Stattdessen wurde beobachtet, dass das Silber in Form von Silberagglomeraten in der TiNb-Ag Beschichtung vorliegt. Mit anderen Worten ist das Silber im Wesentlichen nicht in das TiNb-Gitter integriert. Die Silberagglomerate liegen vorzugsweise in einer Größe in einem Bereich von 1 µm bis 50 µm und noch bevorzugter in einem Bereich von 5 µm bis 30 µm vor.

Des Weiteren wird davon ausgegangen, dass die Wirksamkeit des Silbers insbesondere dadurch entsteht, dass das Silber im implantierten Zustand der Implantatkomponente bei Kontakt mit Körperflüssigkeit durch Lokalelementbildung in den ionischen Zustand übergeht und so seine antimikrobielle Wirkung entfaltet. Diese Lokalelementbildung wird durch die oben beschriebene Anordnung der Silberinseln (Ag-Inseln) an der Oberfläche der Beschichtung ermöglicht. Erreicht wird diese Anordnung durch ein gleichzeitiges Beschichten des Implantats mit Titan, Niob und Silber.

Die Beschichtung weist durch ihre antimikrobiellen Eigenschaften eine infektionshemmende Wirkung auf, die sich insbesondere auch auf Staphylococcus epidermidis bezieht. Es wird vermutet, dass der in der TiNb-Matrix vorliegende Silberanteil der Beschichtung die Ausbildung eines Biofilms stört, den diese Bakterien entwickeln. Durch diese Störung funktioniert der durch diesen Biofilm hervorgerufene Schutzmechanismus der Bakterien vor Antibiotika zumindest nicht mehr ausreichend, sodass diese zurückgedrängt werden können.

Weiterhin wurde beobachtet, dass sich das Silber in Ionenform aus der Beschichtung lösen kann. Es wird davon ausgegangen, dass diese an der Oberfläche der Beschichtung ionisierten Silberpartikel in unmittelbarer Umgebung des Implantats eine Wirkzone ("inhibition zone") ausbilden, in der sie eine antimikrobielle Wirkung entfalten. Folglich kann mit der Beschichtung nicht nur gegen eine sich unmittelbar von der Oberfläche des Implantats ausbreitenden Infektion vorgebeugt werden, sondern auch solche, die sich ansonsten in der Umgebung der Implantatkomponente entwickeln würden.

Eine TiNb-Ag Beschichtung mit einem Silberanteil von 5-30 At% entfaltet eine antimikrobielle Wirkung. Dabei liegt insbesondere eine Wirksamkeit gegen Staphylococcus epidermidis vor. Wie oben beschrieben, ist dieser Erreger üblicherweise auf der Haut von Menschen zu finden und vermutlich deswegen in vielen Fällen Ursache für eine nach der Implantation entstehenden Infektion. Dabei deuten Untersuchungen darauf hin, dass insbesondere im Wirbelsäulenbereich ein erhöhtes Risiko besteht, dass dieser Erreger nach Implantation eine folgenschwere Infektion auslösen kann. Dies wird möglicherweise dadurch begünstigt, dass Staphylococcus epidermidis eine vergleichsweise geringe Virulenz unter den Staphylokokken aufweist. Das führt dazu, dass die Infektionsanzeichen später auftreten und anfangs somit möglicherweise übersehen werden. Da die Beschichtung insbesondere gegen diesen Erreger eine hemmende Wirkung zeigt, kann insbesondere einer aus dem vorgenannten Grund häufig sehr spät erkannten Infektion vorgebeugt werden.

Vorzugsweise ist der At%-Anteil an Silber und/oder der At%-Anteil an Niob jeweils geringer als der At%-Anteil an Titan. Mit anderen Worten ist es nicht erforderlich, dass eine stöchiometrische Verteilung vorliegt. Die Verteilung der Beschichtungskomponenten kann über- oder unterstöchiometrisch sein. In Summe weist die Beschichtung einen Anteil von mindestens 80 At% und insbesondere von mindestens 90 At% TiNb auf. Der At%-Anteil an Titan beträgt vorzugsweise 65-90 At%, 75-85 At% oder in etwa 80 At%.

Durch einen maximalen Silberanteil von 25 At% wird sichergestellt, dass das TiNb Einlagerungen von Silber oder Silberinseln aufweist und nicht umgekehrt. Dies hat den Vorteil, dass das TiNb eine im Wesentlichen durchgehende Beschichtung bereitstellt in der das Silber eingelagert ist. Wie oben beschrieben liegt folglich vorzugsweise kein Bereich auf der Beschichtung vor, bei dem sich eine Silberinsel vollständig durch die Dicke der Beschichtung erstreckt. Ein Herauslösen von Silber hat somit im Wesentlichen keinen negativen Einfluss auf die Funktionalität und Integrität der Beschichtung.

Auch andere bevorzugte Silberanteile für die vorliegende Beschichtung, wie zum Beispiel ein Silberanteil von 1,5-15 At%, 1,5-5 At% oder in etwa 2 At% weisen diesen Vorteil auf. Unter anderem führt dieser Aufbau der Beschichtung dazu, dass insbesondere bei dem weiter unten beschriebenen Beschichtungsverfahren an der Beschichtungsoberfläche zumindest ein Teil des Silberanteils neben dem TiNb-Anteil vorliegt.

Der Silberanteil führt zusammen mit dem TiNb-Anteil als TiNb-Ag Beschichtung neben der oben erwähnten antimikrobiellen Wirkung zudem zu keiner wesentlichen Veränderung der mechanischen Eigenschaften im Verhältnis zu einer reinen TiNb-Beschichtung. So weist sie immer noch eine ausreichend hohe Härte auf, um gegen eine Beschädigung bei der Handhabung des Implantats während der Implantation vorzubeugen und gleichzeitig eine ausreichende Duktilität, welche bei einer elastischen oder plastischen Verformung des Implantats die Integrität der Beschichtung im Wesentlichen nicht beschädigt. Als Ergebnis schützt die TiNb-Ag Beschichtung sowohl vor einer Infektion als auch einem zumindest übermäßigen Austreten von Legierungskomponenten, die anderweitig beim Patienten das Potential haben, eine Überempfindlichkeit hervorzurufen.

Dabei wird vermutet, dass nicht nur die Härte, sondern auch die Duktilität die mechanische Widerstandsfähigkeit bzw. Festigkeit der Beschichtung unterstützt, sodass sie den bei einer Implantation der Implantatkomponente auftretenden mechanischen Einflüssen widersteht. Solche mechanischen Einflüsse entstehen zum Beispiel beim Erzeugen eines Presssitzes einer Implantatkomponente im Knochengewebe, durch Kontakt einer Implantatkomponente mit einem Befestigungselement, wie zum Beispiel beim Eindrehen von Knochenschrauben zur Befestigung einer Platte oder einer Klemmung, oder einer insbesondere plastischen Verformung der Implantatkomponente. Im Bereich der Wirbelsäule treten derlei Belastung zum Beispiel bei der Montage von Implantatkomponente, wie zum Beispiel bei einem Spondylodeseaufbau, auf. Auch bei der Behandlung von Frakturen entstehen während der Implantation solche auf die Implantatkomponente wirkende Belastungen.

Aus diesen Gründen ist die vorliegende Beschichtung insbesondere für Implantatkomponenten geeignet, welche nach Implantation das Skelett eines Patienten unterstützen oder Teile dieses Skeletts ersetzen. Bei solchen Implantatkomponenten tritt eine mechanische Belastung der Beschichtung im Allgemeinen während der Implantation und des Zusammenbaus eines Implantats auf. Nach Implantation entstehen insbesondere durch die alltägliche Belastung des Implantats im Körper eines Patienten Spannungen und Dehnungen in der Beschichtung einer Implantatkomponente. Auch diesen kann die vorliegende Beschichtung widerstehen.

Mit anderen Worten ist die Beschichtung vor allem für Implantatkomponenten geeignet, bei denen Abrieb vor allem während der Implantation und/oder Zusammenbau des Implantats besteht und die Beschichtung im implantierten Zustand im Wesentlichen keinen funktionsbedingten Abrieb erfährt. Es wurde festgestellt, dass hierfür eine Dicke der Beschichtung unter 10 µm, insbesondere von 2,5-6 µm, bevorzugt 3,5-5,5 µm und noch bevorzugter von in etwa 4,5 µm ausreichend ist. Allerdings ist es denkbar, eine derartige Beschichtung auch mit einer höheren Schichtdicke zu verwenden.

Weiterhin kann bei der vorliegenden Beschichtung durch den Silberanteil teilweise der Unterschied der Materialeigenschaften, insbesondere die Elastizität, zu dem darunterliegenden Basismaterial des Implantats verringert werden. Auch dadurch wird eine ausreichende mechanische Widerstandsfähigkeit und Haftung der Beschichtung ermöglicht. Auch kann die Beschichtung unter anderem deswegen auf eine große Vielfalt unterschiedlicher Implantatmaterialien aufgebracht werden, die nicht nur Metalllegierungen, sondern auch Polymere, wie zum Beispiel Polyethylen oder PEEK einschließen. Die vorliegende Beschichtung kann so insbesondere die Widerstandsfähigkeit von aus einem Polymer hergestellten Implantatkomponenten verbessern.

Zusammenfassend zeigt die TiNb-Ag Beschichtung somit sowohl vorteilhafte antimikrobielle als auch vorteilhafte mechanische Eigenschaften, die für ein mit dieser Beschichtung zumindest abschnittsweise beschichtetes Implantat oder Implantatkomponente von großem Nutzen für den Patienten sein kann.

Wie oben bereits beschrieben worden ist, wird eine derartige Beschichtung insbesondere durch Verfahren mit physikalischer Gasabscheidung (PVD-Verfahren) hergestellt.

Hierfür wird die mit der Beschichtung zu versehene Implantatkomponente vor dem Einbringen in die Beschichtungskammer vorzugsweise wässrig gereinigt.

Das Implantat wird in der Beschichtungskammer platziert, die anschließend evakuiert wird. Für die nachfolgenden Prozesse wird das Implantat vorzugsweise auf 400 bis 600 °C aufgeheizt, um die Beweglichkeit von Ionen an der Oberfläche des Implantats zu verbessern und eine bessere Haftung der Beschichtung auf dem Implantat zu erreichen.

Vorzugsweise erfolgt eine Reinigung der Oberfläche der Implantatkomponente innerhalb der Beschichtungskammer und vor dem Aufbringen der Beschichtung. Zum Beispiel kann eine Reinigung mittels einer Glimmentladung in einer Wasserstoffatmosphäre durchgeführt werden, um etwaige organische Rückstände auf der unbeschichteten Implantatoberfläche zu entfernen.

Weiterhin ist es möglich, eine Reinigung der Oberfläche der Implantatkomponente mittels Ionenätzens durchzuführen. Hierbei wird die Implantatkomponente unter einer inerten Atmosphäre, insbesondere einer Argonatmosphäre, mit Ionen (z. B. Titanionen, Argonionen) beschossen, um eine an der Oberfläche des unbeschichteten Implantatmaterials vorhandene Oxidschicht zu entfernen. Auch hierdurch wird eine bessere Haftung der Beschichtung an der Oberfläche des Implantats erreicht.

Jeder der oben genannten Reinigungsschritte findet vorzugsweise in einer Unterdruckatmosphäre von 10-1 bis 10-4 mbar statt.

Nach der optionalen Reinigung durch mindestens einen der oben erläuterten Reinigungsschritte erfolgt das Aufbringen der Beschichtung auf die Implantatkomponente ebenfalls unter einer inerten Atmosphäre, insbesondere einer Argonatmosphäre.

Wie oben bereits beschrieben worden ist, kann diese Beschichtung entsprechend ihrer gewünschten Zusammensetzung mit mindestens einem Silbertarget, mindesten einem Niobtarget und mindestens einem Titantarget ausgeführt werden. Genauso ist es möglich, ein oder mehrere Targets zu verwenden, welche die für die Beschichtung vorgesehenen At%-Anteile an Titan, Niob und/oder Silber aufweist. Mit anderen Worten, können Targets verwendet werden, die mindestens aus zwei der Beschichtungskomponenten bestehen, insbesondere aus dem angestrebten At%-Anteilen von Titan und Silber. Folglich wird dabei die Zusammensetzung der Beschichtung durch die Zusammensetzung des Targets zumindest mitbestimmt.

Um eine Streuung des verdampften Targetmaterials an Gasteilchen in der Beschichtungskammer und damit den Verlust an Targetmaterial möglichst gering zu halten, erfolgt die Beschichtung unter einem Unterdruck in einem Bereich von 10-2 bis 10-3 mbar.

Ist die gewünschte Atmosphäre eingestellt, beginnt der Verdampfungsprozess des mindestens einen Targets. Besonders bevorzugt wird hierfür ein Lichtbogen verwendet, der durch einen starken Strom mittels elektrischer Entladung Material aus den Targets herauslöst und in die Gasphase überführt. Bei dieser Entladung werden insbesondere Spannungen in einem Bereich von 15-30 V und bevorzugt in einem Bereich von 20-25 V sowie Ströme in einem Bereich von 40-70 A eingesetzt. Dem Fachmann ist es allerdings verständlich, dass auch andere Verfahren zum Verdampfen der Targets verwendet werden können, wie zum Beispiel thermisches Verdampfen, Elektronenstrahlverdampfen oder Laserstrahlverdampfen.

Zumindest während eines Teils des Beschichtungsvorgangs erfolgt auch bei Verwendung von mehreren Targets aus unterschiedlichen Materialien die Beschichtung mit diesen Targets gleichzeitig, um die oben beschriebene inselförmige Struktur der TiNb-Ag Beschichtung zu erzeugen.

Je nach zu beschichtendem Basismaterial des Implantats kann an diesem zudem eine negative Spannung von 100 V bis 1500 V angelegt werden, um die Haftung und Schichthomogenität zu verbessern. Auch können, um eine möglichst gleichmäßige Beschichtung zu erreichen, die Targets und das Implantat während des Beschichtungsvorgangs relativ zueinander bewegt werden.

Nach erfolgter Beschichtung und einer Abkühlphase wird die Beschichtungskammer wieder belüftet und das beschichtete Implantat bzw. die beschichteten Implantate können entnommen werden. Die Abkühlung erfolgt vorzugsweise mit Unterstützung einer Gasatmosphäre (z. B. Stickstoff oder einem inerten Gas) für eine verbesserte Wärmeabfuhr, sodass der Abkühlungsvorgang beschleunigt wird.

## Patentansprüche

1. Implantatkomponente, die zumindest abschnittsweise mit einer Beschichtung beschichtet ist, wobei die Beschichtung eine TiNb-Beschichtung ist, die einem At%-Anteil Ti und einen At%-Anteil Nb aufweist, **dadurch gekennzeichnet, dass** die Beschichtung zudem einen At%-Anteil von 1-25 At% Ag aufweist und dass die Implantatkomponente mit der Beschichtung plastisch verformbar ist.

2. Implantatkomponente nach Anspruch 1, wobei die Beschichtung 1,5-15 At% Ag, 1,5-5 At% Ag oder in etwa 2 At% Ag aufweist.

3. Implantatkomponente nach Anspruch 1 oder 2, wobei die Beschichtung 5-40 At% Nb, 10-30 At% Nb, 15-25 At% Nb oder in etwa 18 At% Nb aufweist.

4. Implantatkomponente nach einem der Ansprüche 1 bis 3, wobei auf der Beschichtungsoberfläche Ag und TiNb nebeneinanderliegend ausgebildet sind.

5. Implantatkomponente nach einem der vorstehenden Ansprüche, wobei die Beschichtung eine Dicke von 2,5-6 µm, 3,5-5,5 µm oder in etwa 4,5 µm aufweist.

6. Implantatkomponente nach einem der vorstehenden Ansprüche, bei der die TiNb-Beschichtung im Wesentlichen als nicht stöchiometrische TiNb-Schicht vorliegt.

7. Implantatkomponente nach einem der vorstehenden Ansprüche, wobei die Implantatkomponente eine Knochenplatte oder eine Wirbelsäuleimplantatkomponente, insbesondere eine Komponente eines Spondylodeseimplantats, und vorzugsweise eine Verbindungsstange eines Spondylodeseimplantats ist.

8. Implantatkomponente nach einem der vorstehenden Ansprüche, bei welcher die Oberfläche des beschichteten Abschnitts TiNb mit darin eingebetteten Ag-Inseln aufweist.

9. Implantatkomponente nach einem der vorstehenden Ansprüche, wobei die zu beschichtende Implantatkomponente eine Titanlegierung aufweist und bevorzugt aus dieser besteht.

10. Verfahren zum Herstellen einer Implantatkomponente nach einem der vorstehenden Ansprüche, das die Schritte umfasst:
- Bereitstellen einer zu beschichtenden Implantatkomponente in einer Beschichtungskammer;
- Bereitstellen von mindestens einem Target, sodass bei einem Verdampfen ein vorbestimmtes At%-Verhältnis von Titan, Niob und Silber erzeugt wird;
- Bereitstellen einer inerten Atmosphäre;
- Verdampfen des mindestens einen Targets; und
- gleichzeitiges Beschichten der Implantatkomponente mit dem verdampften Metall des mindestens einen Targets, und
- plastisches Verformen der beschichteten Implantatkomponente vor der Implantation.

11. Verfahren zum Aufbringen einer Implantatbeschichtung nach Anspruch 10, wobei das Verdampfen des mindestens einen Targets durch Lichtbogenverdampfen ausgeführt wird und dabei die an den Targets angelegte Spannung 15-30 V oder 20-25 V und der angelegte Strom 40-70 A betragen.

12. Verfahren nach Anspruch 10 oder 11, bei dem nach dem Bereitstellen des zu beschichtenden Implantats in der Beschichtungskammer eine Reinigung der zu beschichtenden Implantatoberfläche durch eine Glimmentladung unter einer Wasserstoffatmosphäre ausgeführt wird.

13. Verfahren nach einem der Ansprüche 10 bis 12, bei dem nach dem Einbringen des zu beschichtenden Implantats in die Beschichtungskammer eine Reinigung der zu beschichtenden Implantatoberfläche durch Beschießen der Implantatoberfläche mit Ionen unter einer inerten Atmosphäre durchgeführt wird.

14. Verwendung einer Beschichtung, wobei die Beschichtung eine TiNb-Beschichtung ist, die einem At%-Anteil Ti und einen At%-Anteil Nb aufweist, und wobei die Beschichtung zudem einen At%- Anteil von 1-25 At% Ag aufweist, für die Herstellung einer Implantatkomponente die plastisch verformbar ist.

## Claims

1. Implant component of which at least portions are coated with a coating, the coating being a TiNb coating having an at% fraction of Ti and an at% fraction of Nb, **characterized in that** the coating also has an at% fraction of 1-25 at% of Ag, and **in that** the implant component with the coating is plastically deformable.

2. Implant component according to Claim 1, wherein the coating has 1.5-15 at% of Ag, 1.5-5 at% of Ag or approximately 2 at% of Ag.

3. Implant component according to Claim 1 or 2, wherein the coating has 5-40 at% of Nb, 10-30 at% of Nb, 15-25 at% of Nb or approximately 18 at% of Nb.

4. Implant component according to any of Claims 1 to 3, wherein Ag and TiNb are adjacent to one another on the coating surface.

5. Implant component according to any of the preceding claims, wherein the coating has a thickness of 2.5-6 µm, 3.5-5.5 µm or approximately 4.5 µm.

6. Implant component according to any of the preceding claims, in which the TiNb coating is present substantially in the form of a non-stochiometric TiNb layer.

7. Implant component according to any of the preceding claims, wherein the implant component is a bone plate or a spinal column implant component, more particularly a component of a spondylodesis implant, and preferably a connecting rod of a spondylodesis implant.

8. Implant component according to any of the preceding claims, in which the surface of the coated portion comprises TiNb with Ag islands embedded therein.

9. Implant component according to any of the preceding claims, wherein the implant component to be coated comprises a titanium alloy and preferably consists of said alloy.

10. Process for producing an implant component according to any of the preceding claims, comprising the steps of:
- providing an implant component to be coated in a coating chamber;
- providing at least one target, so that vaporization is accompanied by generation of a predetermined at% ratio of titanium, niobium and silver;
- providing an inert atmosphere;
- vaporizing the at least one target; and
- simultaneously coating the implant component with the vaporized metal of the at least one target, and
- plastically deforming the coated implant component prior to implantation.

11. Process for applying an implant coating according to Claim 10, wherein the vaporization of the at least one target is performed by arc vaporization and the voltage applied to the targets is 15-30 V or 20-25 V and the current applied is 40-70 A.

12. Process according to Claim 10 or 11, in which, after the providing of the implant to be coated in the coating chamber, the implant surface to be coated is cleaned by glow discharge under a hydrogen atmosphere.

13. Process according to any of Claims 10 to 12, in which, after the implant to be coated has been introduced into the coating chamber, the implant surface to be coated is cleaned by bombardment of the implant surface with ions under an inert atmosphere.

14. Use of a coating, the coating being a TiNb coating which has an at% fraction of Ti and an at% fraction of Nb, and the coating also having an at% fraction of 1-25 at% of Ag, for producing an implant component which is plastically deformable.

## Revendications

1. Composant d'implant qui est recouvert au moins par portions d'un revêtement, le revêtement étant un revêtement de TiNb ayant une teneur en % atomique de Ti et une teneur en % atomique de Nb, **caractérisé en ce que** le revêtement a également une teneur en % atomique de 1 à 25 % atomiques d'Ag, et **en ce que** le composant d'implant pourvu du revêtement est déformable plastiquement.

2. Composant d'implant selon la revendication 1, le revêtement comportant 1,5 à 15 % atomiques d'Ag, 1,5 à 5 % atomiques d'Ag ou environ 2 % atomiques d'Ag.

3. Composant d'implant selon la revendication 1 ou 2, le revêtement comportant 5 à 40 % atomiques de Nb, 10 à 30 % atomiques de Nb, 15 à 25 % atomiques de Nb ou environ 18 % atomiques de Nb.

4. Composant d'implant selon l'une des revendications 1 à 3, Ag et TiNb étant formés côte à côte sur la surface du revêtement.

5. Composant d'implant selon l'une des revendications précédentes, le revêtement ayant une épaisseur de 2,5 à 6 µm, 3,5 à 5,5 µm ou d'environ 4,5 µm.

6. Composant d'implant selon l'une des revendications précédentes, le revêtement de TiNb étant présent essentiellement sous la forme d'une couche de TiNb non stœchiométrique.

7. Composant d'implant selon l'une des revendications précédentes, le composant d'implant étant une plaque osseuse ou un composant d'implant rachidien, en particulier un composant d'un implant de spondylodèse, et de préférence une tige de liaison d'un implant de spondylodèse.

8. Composant d'implant selon l'une des revendications précédentes, la surface de la portion revêtue comprenant du TiNb avec des îlots d'Ag incorporés à l'intérieur.

9. Composant d'implant selon l'une des revendications précédentes, le composant d'implant à revêtir comprenant une alliage de titane et de préférence étant en celui-ci.

10. Procédé de fabrication d'un composant d'implant selon l'une des revendications précédentes, ledit procédé comprenant les étapes suivantes :
- fournir un composant d'implant à revêtir dans une chambre de revêtement ;
- fournir au moins une cible de sorte qu'un rapport en % atomique prédéterminé de titane, de niobium et d'argent soit produit lors de la vaporisation ;
- fournir une atmosphère inerte ;
- vaporiser l'au moins une cible ; et
- revêtir en même temps le composant d'implant avec le métal vaporisé de l'au moins une cible, et
- déformer plastiquement le composant d'implant revêtu avant l'implantation.

11. Procédé d'application d'un revêtement d'implant selon la revendication 10, la vaporisation de l'au moins une cible étant effectuée par vaporisation à l'arc, la tension appliquée aux cibles étant de 15 à 30 V ou de 20 à 25 V et le courant appliqué étant de 40 à 70 A.

12. Procédé selon la revendication 10 ou 11, la surface de l'implant à revêtir étant nettoyée par une décharge luminescente sous atmosphère d'hydrogène après que l'implant à revêtir a été disposé dans la chambre de revêtement.

13. Procédé selon l'une des revendications 10 à 12, la surface de l'implant à revêtir étant nettoyée par bombardement de la surface de l'implant avec des ions dans une atmosphère inerte après que l'implant à revêtir a été introduit dans la chambre de revêtement.

14. Utilisation d'un revêtement, le revêtement étant un revêtement de TiNb ayant une teneur en % atomique de Ti et une teneur en % atomique de Nb, et le revêtement ayant en plus une teneur en % atomique de 1 à 25 % atomiques d'Ag pour produire un composant d'implant qui est déformable plastiquement.
